Europäisches Patentamt

European Patent Office

Office européen des brevets

(11) Publication number: **0 463 570 A1**

# EUROPEAN PATENT APPLICATION

(21) Application number: **91110170.7**

(22) Date of filing: **20.06.91**

(51) Int. Cl.5: **C12Q 1/66**, C12N 15/85, C12Q 1/70, G01N 33/569

(30) Priority: **22.06.90 IL 94834**
**25.01.91 IL 97047**

(43) Date of publication of application:
**02.01.92 Bulletin 92/01**

(84) Designated Contracting States:
**AT BE CH DE DK ES FR GB GR IT LI LU NL SE**

(71) Applicant: **R.O.B.I.T. RESEARCH AND DEVELOPMENT COMPANY LTD., c/o EFRATI, GALILI & CO.**
**19 Ahad Haam Str.**
**Tel-Aviv 65541(IL)**

(72) Inventor: **Honigman, Alexander**
**25 Kobobi St. Ramot Denya**
**Jerusalem 96757(IL)**
Inventor: **Israel, Shoshana**
**88 Keren Kayemet Str.**
**Jerusalem 92465(IL)**
Inventor: **Ulitzur, Shimon**
**3 Biram St.**
**Haifa 34986(IL)**
Inventor: **Kuhn, Jonathan C.**
**44 Shearit Hapleta**
**Haifa 34987(IL)**
Inventor: **Suissa, Mordechai**
**Givat Bet Hakerem 4/23**
**Jerusalem 96268(IL)**
Inventor: **Bentwitch, Zvi**
**6 Hagra St.**
**Rehovot 76310(IL)**

(74) Representative: **Vossius & Partner**
**Siebertstrasse 4 P.O. Box 86 07 67**
**W-8000 München 86(DE)**

(54) **Detection of HIV infection by bioluminescence.**

(57) A method for the diagnosis of HIV infection is provided which is based on the use of tester cells, prepared by genetic engineering, which are capable of expressing an enzyme such as luciferase, which catalyses a reaction by which light is emitted, in the presence of either HIV particles and/or the HIV-TAT. In accordance with this method the tester cells are incubated with a blood sample or a fraction thereof, and light detected, in either the living cells or in extracts above background levels, indicating an HIV infection.

d. pZipHIVLUC I

e. pZipHIVLUC 2

f. pZipΔHIVLUC 4

Fig.4b

The present invention concerns a method, kit and system for the detection of HIV infection in suspected subjects. In accordance with the method of the present invention, genetically engineered cells capable of emitting light when exposed to agents present in blood samples of HIV infected subjects, are utilized.

The present invention also provides recombinant DNA molecules suitable for transfecting cells to have the aforementioned light emission capabilities and furthermore provides such transfected cells.

Since the first diagnosis of acquired immuno-deficiency syndrome (AIDS) in the beginning of the 1980's and the subsequent characterisation of the disease and its causative agent, HIV, one of the main goals of AIDS research has been to develop reliable assays for the early diagnosis of HIV infection. Such assays are of particular importance for the prevention of an epidemic spread of the disease.

Various assays for the diagnosis of AIDS have been hitherto available. According to one of these assays, positive diagnosis of AIDS is based upon the determination of HIV antigens in blood samples by immunological means employing anti-HIV antibodies. However, as HIV antigens are usually only present in minute quantities in blood samples, their direct detection is very difficult. Additionally, various HIV strains have been identified which often differ significantly from one another antigenically, thus making their identification by immunological methods very difficult. This immunological assay has another drawback in that a certain level of cross-reactivity exists between HIV-related and non-HIV-related antigens with respect to the anti-HIV antibodies, which may give rise to false positive results.

Another widely used assay is based on the detection of antibodies directed against HIV in the blood of suspected subjects. However, for the aforementioned reasons, one of the major drawbacks of such a method resides in the fact that due to the existence of various HIV strains, different types of antibodies may be produced by different infected individuals. Further, due to the cross-reactivity between HIV-related and HIV non-related antigens, antibodies in blood samples which are shown to recognize HIV antigens in the assay, may have been elicited to non-HIV antigens in the suspected individuals, thus giving rise to false-positive results.

Furthermore, the above-noted assays have additional serious drawbacks by virtue of their being indirect. For one, many HIV-infected individuals do not develop anti-HIV antibodies, and this is particularly the case with infants of up to six months of age, with the consequence that many false-negative results may be obtained from these assays. Additionally, the appearance of anti-HIV antibodies in infected individuals usually takes several weeks and, in some individuals, even several months to years. Finally, due to the above-noted drawbacks, a definite diagnosis of AIDS usually requires considerable time and may take up to three months, the implication of this being that infected individuals may unwittingly continue to infect others.

It is thus clear that both the above antigen and antibody detection assays may give rise to a relatively large proportion of false positive and false negative results.

Against this background, it is not surprising that many attempts have been concentrated on developing direct methods to detect the presence of HIV in the blood of suspected subjects. One such method hitherto proposed is the Polymerase Chain Reaction (PCR). By this method, the DNA present in a clinical sample is purified, and by a rather complex procedure the presence or absence of one HIV DNA sequence is determined. However, in addition to its technical complexity, requiring skilled laboratory personnel not usually employed in standard clinical laboratories, this method was found to be unreliable, giving rise at times to false positive results. Furthermore, this method is incapable of distinguishing between infective and non-infective viruses.

A method for the direct detection of HIV infectious particles was also proposed in the international patent application WO 89/03878. However, the method disclosed by this patent application is merely theoretical since no experimental results have been described and by the experience and knowledge gained during development of the present invention this method is believed to be non-workable.

It is the object of the present invention to provide a method for the diagnosis of an HIV infection which overcomes the above-noted deficiencies of the prior art. More particularly, it is the object of the present invention to provide a method for the direct determination of HIV infection by assaying a blood sample for the presence of HIV infectious particles, HIV infected $T_4$ helper cells, or the HIV inducer protein - TAT.

Another object of the present invention is to provide recombinant DNA molecules and cells transfected therewith useful in said method.

A further object of the present invention is to provide a kit and a system for carrying out the above method.

In the following description reference will at times be made to various prior art documents by indicating their number from the list of references which is to be found at the end of the description before the claims.

The replication of the HIV retrovirus genome is controlled by a promoter which is present in the LTR (long terminal repeat) sequence (**1**, **3**, **4**) and is induced by the regulatory protein TAT, which is encoded by the retroviral gene *tat* (**2**, **3**, **4**). The TAT protein, as well as the tar site, are highly conserved in different HIV strains. Upon HIV infection, TAT is immediately produced in infected cells and thus, if found in a sampler it serves as a clear indication of the presence of HIV in the sample. However, since TAT is usually present in minute quantities, its direct detection, e.g. by immunological means, is very difficult.

In accordance with the present invention, HIV infections are diagnosed by the use of cells transfected with a recombinant DNA molecule comprising a heterologous DNA sequence which encodes an enzyme capable of catalysing a reaction by which light is emitted, expression of said sequence being under the expression control of a TAT-inducible HIV promoter. A suitable DNA sequence is one encoding the enzyme luciferase (of firefly origin) which catalyses an ATP-dependent reaction by which light is emitted during the degradation of the substrate luciferin. In the presence of TAT protein within the transfected cell, to be referred to hereinafter as **tester cells**, the enzyme, being under the expression control of the *tar* site, will be expressed. By subjecting these cells or extracts thereof to appropriate conditions light will be emitted, indicating the existence of an HIV *tat* product.

The present invention thus provides a direct method for the diagnosis of HIV infection in a suspected individual, comprising:

a) providing tester cells transfected by a recombinant DNA molecule having

i) an HIV TAT-inducible promoter selected from the group consisting of an HIV-LTR or part thereof including the *tar* site or an analogous sequence capable of inducing the expression of a downstream DNA sequence in the presence of HIV TAT, and

ii) a heterologous DNA sequence encoding an enzyme capable of catalysing a reaction by which light is emitted, said sequence being under the expression control of said promoter,

said tester cells being capable of absorbing a TAT protein from a surrounding medium and/or susceptible to HIV infection;

b) contacting said tester cells with a blood sample from the suspected individual or with an appropriate fraction thereof and subjecting the tester cells or extracts thereof to conditions in which expression of said heterologous DNA sequence will result in light emission; and

c) monitoring light emission by said cells or extracts thereof, the detection of light emission above background levels indicating an HIV infection.

Two embodiments of the method of the present invention are provided. By one embodiment, to be referred to hereinafter as the **infection embodiment**, the tester cells are susceptible to HIV infection. The susceptibility to HIV infection requires the presence of at least the CD4 receptor antigen on the surface of cells, thus cells to be used in accordance with this embodiment display the CD4 receptor antigen on their surface. Such cells may, for example, be $T_4$ helper cell lines transfected with said DNA molecules or other cells cotransfected with said DNA molecules and a gene encoding the CD4 receptor so as to render them susceptible to HIV infection. CD4 displaying cells may be infected with HIV either by way of free HIV particles or by fusion with cells already infected by the virus. Thus, the tester cells in accordance with the infection embodiment, will be incubated with a blood sample, or a suitable fraction thereof, containing either the sample's $T_4$ helper cells (which are the cells in the blood predominantly infected with HIV), the free HIV particles, if present in the blood, or both. If the blood sample contains either free HIV particles or HIV infected $T_4$ helper cells, some of the tester cells will be infected and as a result TAT will be produced in the cells and the enzyme catalysing the light-emitting reaction will be expressed.

By another embodiment of the present invention, to be referred to hereinafter as the **transactivation embodiment**, the tester cells have the ability to absorb TAT protein from their surrounding medium. Many cells tested to date were found to have this ability (**5** and tests carried out in accordance with the invention) and may thus be used for preparing tester cells by transfection in accordance with this embodiment of the invention. The cells, in accordance with this embodiment, are thus incubated either with a blood sample or fractions thereof containing the TAT protein if present in the sample. Since TAT is a nuclear protein, namely present predominantly in cells' nuclei, it is preferred in accordance with this embodiment to lyse the cells of the sample so that the content of TAT in the HIV infected cells will be released to the medium. This lysate will then be incubated with the tester cells and if TAT is present it will be absorbed by the tester cells inducing the expression of the enzyme catalysing the light-emitting reaction.

In order to monitor the expression of the said enzyme, in accordance with both above embodiments, either the tester cells or their extracts are subjected to reaction conditions in which light will be emitted. For example, where the enzyme is luciferase, said conditions are the provision of luciferin and an appropriate temperature, and where the light emissions are tested on tester cell extracts also ATP. Light emission will then be monitored, and levels thereof above background levels will indicate the presence of HIV in the tested sample.

The present invention also provides a recombinant DNA molecule for use in the above method, comprising an HIV promoter being an HIV-LTR or part thereof including the *tar* site or an analogous sequence capable of inducing the expression of a downstream DNA sequence in the presence of HIV TAT, and a heterologous DNA sequence encoding an enzyme catalysing a light-emitting reaction, said DNA sequence being under the expression control of said promoter. Particularly preferred, in accordance with the present invention, are recombinant DNA molecules wherein said heterologous DNA sequence encodes the enzyme luciferase.

The present invention also provides cells transfected by said recombinant DNA molecules which are useful in said method.

The present invention also provides a process for preparing said tester cells, comprising either:

i) transfecting cells which are capable of absorbing a TAT protein from their surrounding medium with said recombinant DNA molecule;

ii) transfecting cells susceptible to HIV infection with said recombinant DNA molecule; or

iii) transfecting cells with said recombinant DNA molecule and at the same time or subsequently transfecting the cells with a gene encoding the CD4 receptor so as to render the cells capable of being infected by HIV.

Finally, the present invention also provides kits and systems for carrying out the method of the invention.

The present invention provides a novel approach in the detection of HIV infections in suspected subjects. The use of marker genes such as *cat* (encoding an HIV envelope protein) in assays for the detection of the TAT proteins has been known in the art (**6**, **7**). By such known assays the determination of the activation of the marker genes was carried out *in vitro* in cell extracts employing various radioactive substances. In addition to the undesirable use of radioactive substances, such assays are not very sensitive. In contrast to such hitherto use of marker genes, the measurement of light in accordance with the present invention is very sensitive, since with suitable and relatively inexpensive instrumentation, which is within the reach of most clinical laboratories, even a few photons can be measured. Light detection may be carried out in any available photometer, such as, for example, the photo intensified charge coupled device (CCD), which enables the measurement of light from single cells and the reduction to a minimum (by electronic subtraction means) of the background "noise". Alternatively, light detection may be performed by direct exposure of a tester cell preparation or extracts thereof to a photographic film. (For the manner of light detection see also below).

The high sensitivity of the method in accordance with the present invention allows the detection of even a few viable viral particles in the tested sample, and thus false negative results are reduced to minimal proportions.

For the preparation of tester cells, cells are transfected with the said recombinant DNA molecule comprising the HIV-LTR *tar* site and a heterologous DNA sequence encoding an enzyme, such as luciferase, which is capable of catalysing a light-emitting reaction, said sequence being under the expression control of the *tar* site. Cells to be transfected are preferably human cell lines and particularly preferred is the human kidney cell line - 293 (**8**).

Tester cells for use in the infection embodiment of the present invention have to be susceptible to HIV infection and thus they must display at least the CD4 antigen on their external surface. Such tester cells may be prepared, for example, from $T_4$ helper cell lines or other cell lines displaying the CD4 antigen and being susceptible to such an infection. Further, cell lines not usually displaying the CD4 antigen, but susceptible to HIV infection after transfection with a CD4 gene, may also be used in accordance with this embodiment, such CD4 transfection to be effected prior to, at the same time or after transfection of the cells with the enzyme encoding recombinant DNA molecules. For example, where the 293 cell line is used, which does not display CD4 antigens on its external surface, the cells must also be transfected with another recombinant DNA molecule comprising a CD4 gene.

Tester cells for use in the transactivation embodiment of the present invention have to be capable of absorbing the TAT proteins from the surrounding medium if present. Several cell lines tested to date were found to have this capability and may thus be used for the preparation of tester cells in accordance with this embodiment. The 293 cell line is capable of absorbing the TAT protein from the surrounding medium and may thus be transfected with the said enzyme encoding recombinant DNA molecule to obtain tester cells useful in accordance with this embodiment.

For assaying HIV infection in accordance with the infection embodiment, the tester cells are subjected to an infection stage in which they are incubated together with a blood sample, or with an appropriate fraction thereof, which contains the $T_4$ lymphocytes, free HIV particles if such are present in the sample, or both. If the tested sample was obtained from an HIV infected subject, it will contain either free HIV particles or $T_4$ infected lymphocytes, or both, and by contacting such a sample or a said fraction thereof, some of the tester cells will be infected with the virus. Such an infection involves the introduction of the viral genetic material into the infected cell of a quantity of the TAT protein as well as the viral genetic material. As the viral genom encodes *inter alia* also the TAT protein, this protein will now be expressed in the infected cell. Consequently, the so-produced TAT protein will transactivate the HIV promoter in said DNA molecule an consequently the enzyme which catalyses light production will be expressed. As a result, under appropriate conditions, light will be generated. The manner of monitoring light emission is explained further below.

In accordance with the transactivation embodiment, the tester cells are subjected to a transactivation stage in which they are brought into contact with a blood sample or a fraction thereof which contains the TAT protein if present in the tested sample. The TAT protein is present in minute quantities in the blood of infected subjects, but as it is a nuclear protein it is preferable in accordance with this embodiment to lyse the $T_4$ helper cells, which are the cells in the blood which are infected by HIV. Following lysis these cells will release their content of the TAT protein into the medium. Thus, preferably, either the lysate or protein-containing fractions thereof are brought into contact with the tester cells. If the tested sample is from an HIV infected individual, TAT will be present and its absorption into the tester cells will result in the expression of said enzyme. Similarly as in accordance with the infection embodiment, the activity of the enzyme is assayed by monitoring light emission.

The two embodiments of the method in accordance with the invention may be combined by the use of tester cells capable of being both infected by HIV and of absorbing the TAT protein from the surrounding medium. Such a tester cell may, for example, be a tester cell derived from the 293 cell line and which was also transfected with a CD4 gene.

The efficacy of the method in accordance with the infection embodiment of the present invention may be increased by the addition of a fusing or a transport facilitating agent during the infection stage, such as polyethylene glycol (PEG), polybrene, DEAE dextran and many others which are known *per se*.

The expression of the enzyme catalysing the light-emitting reaction is assayed by monitoring light emission under appropriate conditions. For such monitoring, either the whole tester cells or their extracts are incubated with appropriate substrates under appropriate incubation conditions for light emission during the reaction catalysed by said enzymes. For example, where the enzyme is luciferase, such conditions require the addition of luciferin, and where the light emission is monitored on tester-cell extracts, also the addition of ATP.

It is preferred to compare the results with control results obtained from tester cells incubated under similar conditions with a control sample. Increase of light production above control levels will indicate the presence of HIV in the tested sample. Additionally, the amount of emitted light may be compared to the amount of light obtained with various standards.

The recombinant DNA molecule of the present invention is capable of expressing an enzyme which catalyses a reaction by which light is emitted, which expression is induced by the HIV inducer protein TAT. This molecule comprises a DNA sequence encoding said enzyme and an HIV TAT-inducible promoter selected from the group consisting of said HIV-LTR or part thereof including the *tar* site, which is the TAT protein recognition site, or analog sequences capable of inducing expression of a downstream DNA sequence in the presence of HIV TAT, the enzyme-encoding sequence being under the expression control of the promoter. Particularly preferred is a DNA sequence encoding the firefly luciferase which catalyses an ATP dependent reaction by which light is emitted during degradation of the substrate luciferin.

A polyadenylating (poly A) site and signal sequence at its 3' end are required for generation of mRNA. Seeing that the poly A site and signal of the luciferase gene are inefficient, it is preferable to include in the recombinant DNA molecules of the present invention, comprising the luciferase encoding sequence, a different poly A signal and site, such as that of SV40. The recombinant DNA molecule in accordance with the invention preferably also comprises a marker gene, such as that conferring antibiotic resistance, which facilitates the identification of cells transfected thereby.

The HIV-LTR promotes some expression of the said heterologous DNA sequence even in the absence of TAT. This will thus increase the background signal in the above method and thus reduce its sensitivity. During the course of development of the present invention, it was realized that this background expression may be reduced to minimal levels by promoter occlusion, namely placing the HIV LTR downstream to another promoter, which is more active than the HIV promoter, in the absence of TAT. When TAT is present, the activity of the HIV promoter increases several-fold, thus overcoming this occlusion.

Accordingly, by a preferred embodiment the HIV LTR in the DNA molecule is downstream to another promoter which is more active than the HIV promoter in the absence of TAT.

In accordance with an even more preferred embodiment of the invention, HIV directed transcription is in an antisense direction relative to the said other promoter. In such a DNA molecule the signal to noise ratio will increase even more.

The kits in accordance with the invention will comprise the ingredients necessary for carrying out the method of the present invention. These ingredients include transfected cells, for use with either the infection or the transactivation embodiment. Additionally, such kits may also include appropriate incubation media for the cells, a substrate for the enzyme, such as luciferin where the enzyme is luciferase, buffers for carrying out the light reaction, etc. Optionally, the kit may also comprise various standards, e.g. a sample containing a known concentration of the TAT protein.

A system for carrying out the method according to the invention will comprise the same ingredients as in the above kits, with the addition of a light monitoring device.

The following description exemplifies recombinant DNA molecules, transfected cells and tests carried out in accordance with the present invention. These examples should be construed as an illustration of the present invention and should not be considered limiting.

In the following description, reference will at times be made to the accompanying drawings, in which:

**Fig. 1** is a schematic representation of the manner of preparation of pALUCAT which has a *luc* encoding DNA sequence under the expression control of HIV-LTR;

**Fig. 2** is a schematic representation of the manner of preparation of pZipHIVLUC plasmids which have *luc* gene under the expression control HIV-LTR which is downstream to an MLV-LTR;

**Fig. 3** is a schematic representation of the manner of preparation of pZipΔHIVLUC, which are plasmids similar to the plasmids prepared in accordance with Fig. 2 but differ in that the HIV-LTR lacks 160 base pairs;

**Fig. 4** shows in detail the HIV-LTR/luc region of several plasmids used in the experiments below, both plasmids in which the HIV LTR is not occluded (a) and those in which the HIV LTR is occluded by another promoter (b);

**Fig. 5** shows light production by 293 cells transiently transfected with either the plasmid pZipΔHIVLUC4 (a) or pALUCAT (b);

**Fig. 6** shows results of dot-hybridization of a *tat* specific DNA probe to RNA obtained from three clones of 293 cells transfected with pRSVTAT (3 upper spots), a positive control with the pRSVTAT plasmid (lower center spot) and a negative control with RNA obtained from mock transfected 293 cells (lower right corner);

**Fig. 7** shows light production from growing cells of two of the clones in Fig. 6 transfected with the plasmid pZipΔHIVLUC4: a and c - 5 hours of exposure to a light sensitive film; b and d - 17 hours exposure to a light sensitive film;

**Fig. 8** shows light production by 293 growing cells transfected with pZipΔHIVLUC4 exposed to extracts from the three pRSVTAT transfected clones and the mock transfected clone of Fig. 6;

**Fig. 9** shows light production by 293 cells stably transfected with pZipΔHIVLUC4 plasmid exposed to extract of either normal 293 cells (a) or extracts of TAT transfected 293 cells (b), after an overnight exposure;

**Fig. 10** shows light emission by pelleted 293 cells transfected with pZipΔHIVLUC4 and exposed to extracts of TAT transfected cells (exposure time - 2 minutes); and

**Fig. 11** shows light production by growing 293 cells, stably transfected with the plasmid pZipΔHIVLUC4, exposed to various dilutions of extracts of HIV infected cells.

## PREPARATION OF RECOMBINANT DNA MOLECULES

In the following, the preparation of plasmids prepared in accordance with the present invention is described. DNA cleavage and ligation of the pertinent DNA fragments to construct the plasmids was performed in accordance with methods known *per se*.

At each stage, the constructed plasmids were cloned into E. coli DR 100 cells and the transfected cells were selected for conferred ampicillin resistance by an Amp$^R$ gene carried by all the plasmids prepared. After selection as specified, plasmids were purified from each of approx. 50 of the selected colonies and restriction analysis of these plasmids was carried out. After such an analysis, one clone was selected, grown, and its plasmids purified, which then served for subsequent stages of plasmid construction or for transfection of tester cells, where appropriate.

### a. Preparation of pALUCAT

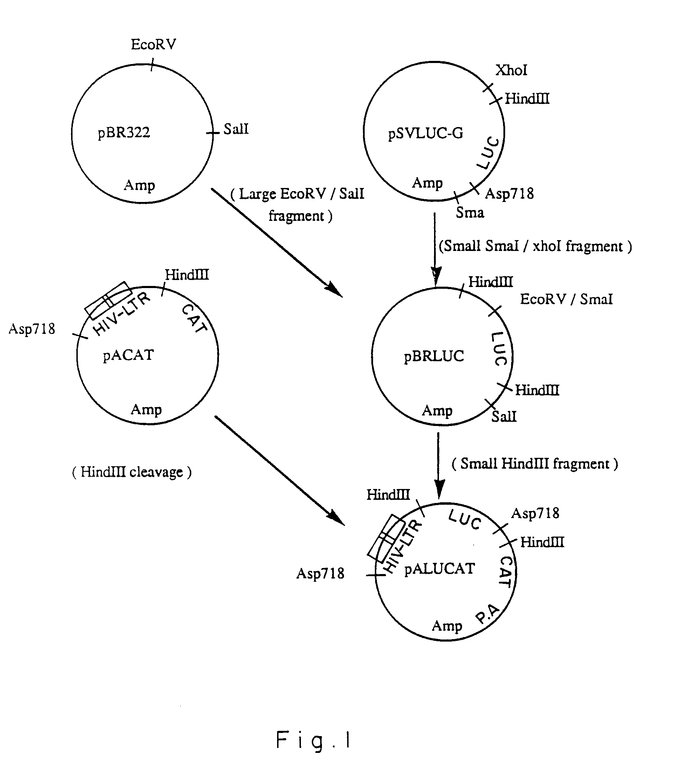

A schematic description of the construction of the plasmid pALUCAT is shown in Fig. 1. The source of the *luc* gene (encoding the enzyme luciferase) was the plasmid pSVLUC-G obtained from J. Collins, FRG. The *luc* gene was isolated and purified following cleavage of pSVLUC-G with SmaI and XhoI restriction enzymes. The plasmid pBR322 (**9**) which carries a gene for ampicillin resistance, was cleaved by SalI and

by EcoRV restriction enzymes, the small 466 b.p. fragment discarded, and the purified *luc* fragment from pSVLUC-G was ligated in its place, to give rise to the plasmid pBRLUC.

As illustrated in Fig. 1, the *luc* gene fragment obtained from pSVLUC-G has a single HindIII restriction site close to the XhoI restriction site. The plasmid pBR322 also contains a single HindIII restriction site on the large EcoRV-SalI fragment close to the EcoRV site. Thus, the *luc* gene in pBRLUC is flanked by HindIII restriction sites. pBRLUC was thus cleaved with HindIII and the *luc* gene fragment so obtained was ligated into the HindIII restriction site of pACAT (A. Panet et al., Biotechnology General-BTG, Nes Ziona, Israel). The plasmid harbours an HIV-LTR fused to and upstream of the *cat* gene.

The resulting pALUCAT plasmid thus comprises the *luc* gene under the expression control of HIV-LTR. This plasmid also comprises the *Amp* gene of pACAT which confers ampicillin resistance.

The pALUCAT plasmid so constructed is suitable for use in transfecting cells to be used as tester cells in accordance with the invention.

The degree of light emission in the pALUCAT carrying tester cells depends on the degree of activation of the HIV-LTR. In order to obtain a proper calibration, cells belonging to the same cell line as the pALUCAT containing cells were transfected by the plasmid pACAT. A calibration is thus obtained by comparing the degree of light emission in the pALUCAT transfected cells to the degree of the CAT production in the pACAT transfected cells.

It should be noted that the HIV-LTR expresses some background level activity even in the absence of the TAT protein. Thus, some degree of expression of the *luc* gene occurs continuously even in the absence of the TAT protein and therefore, when used in accordance with the method of the invention, cells containing this plasmid show a certain degree of background bioluminescence.

### b. Construction of pZipHIVLUC

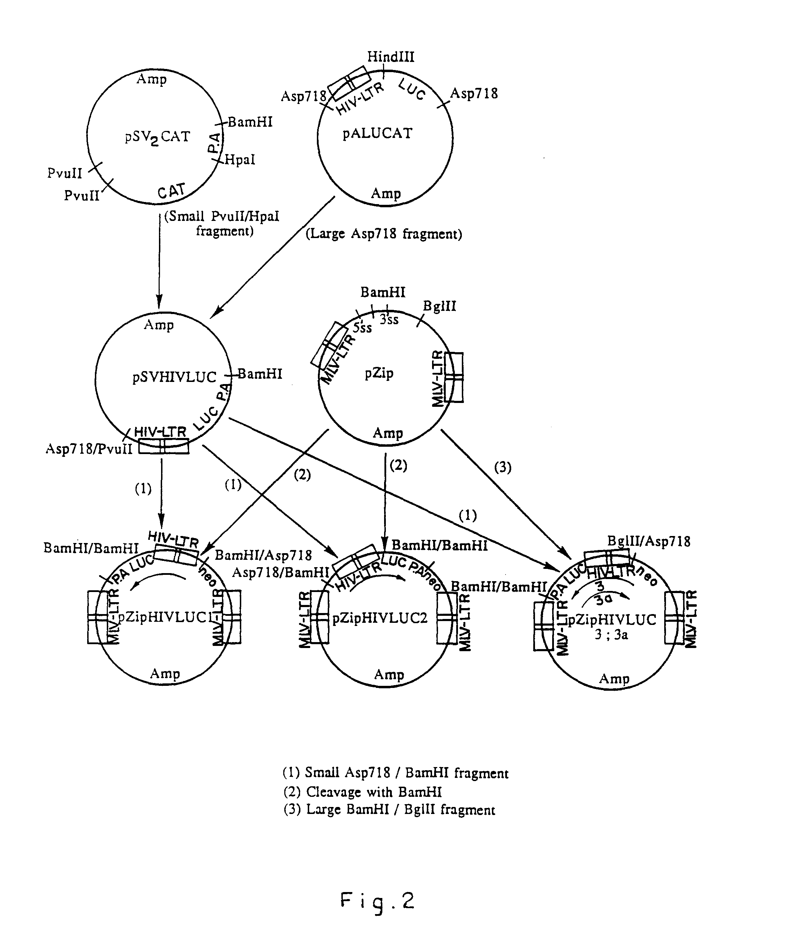

A schematic representation of the construction of the pZipAIVLUC plasmids is shown in Fig. 2.

The pALUCAT plasmid has two Asp718 restriction sites (as shown in Fig. 1) flanking the full HIV-LTR sequence and the *luc* gene. After cleavage of pALUCAT with Asp718, the DNA fragments containing the HIV-LTR and the *luc* gene were isolated and purified and the cohesive ends thereof were filled. The plasmid $pSV_2CAT$ (**10**) was cleaved with PvuII and HpaI restriction enzymes, and the fragment containing the *cat* gene was discarded. The cohesive ends of the remaining part of $pSV_2CAT$ were filled as above, and this fragment was then ligated to the said DNA fragment from pALUCAT. Such a ligation reconstitutes the PvuII restriction site and the Asp718 restriction site attached thereto, while the PvuII and the other Asp718 restriction sites are lost.

The resulting pSVHIVLUC plasmid has the HIV-LTR, the *luc* gene and the SV40 polyadenylation signal (P.A. in Fig. 2) between an Asp718 and a BamHI restriction sites.

The plasmid pZip (**11**) harbours the prokaryotic *bla* gene, which confers ampicillin resistance and an origin of replication of pBR327. The neomycin resistance conferring gene (neo[R]) and the MLV packaging signal are flanked by MLV-LTR DNA sequences. This plasmid has a unique BamHI restriction site between the donor and acceptor splice sites close to the 5' LTR allowing expression of a cloned gene at the BamHI site, as well as the expression of the neo[R] gene.

The DNA fragment located between the Asp718 and BamHI cleavage sites in plasmid pSVHIVLUC, which includes the HIV-LTR and the *luc* gene as well as the poly A signal and site, was isolated. The fragment was ligated to the BamHI cleavage site of pZip, the incompatible cohesive ends were filled in and religated.

Two types of plasmids were produced in this manner, in which the HIV-LTR is occluded by the upstream MLV-LTR:

1) pZipHIVLUC1, in which the HIV-LTR/*luc*/PolyA DNA fragment was inserted in an antisense orientation relative to the MLV-LTR directed transcription, namely in an orientation in which the MLV-LTR directed transcription and the HIV directed transcription are in opposite directions.

2) pZipHIVLUC2, in which the HIV-LTR/*luc*/Poly A fragment was inserted in a sense orientation relative to MLV-LTR directed transcription.

A different set of plasmids was prepared by inserting the HIV-LTR *luc* DNA fragments from pSVHIVLUC between the BamHI and the BglII restriction sites of pZip. Two constructs were thus obtained:

(1) pZipHIVLUC3, in which the orientation of the HIV-LTR/*luc*/PolyA DNA fragment is the same as that of pZipHIVLUC1.

(2) pZipHIVLUC3a, in which the orientation of the HIV-LTR/*luc*/PolyA DNA fragment is the same as that of pZipHIVLUC2.

In pZipHIVLUC3 and 3a, the 3' splice site is deleted facilitating expression of all the HIV-LTR derived transcripts, since there is no splicing out of the sequences. These two plasmids also do not express the neo[R] gene, and thus selecting tester cells transfected by these plasmids is conveniently carried out by cotransfection with PSV2NEO.

c. Preparation of pZipΔHIVLUC

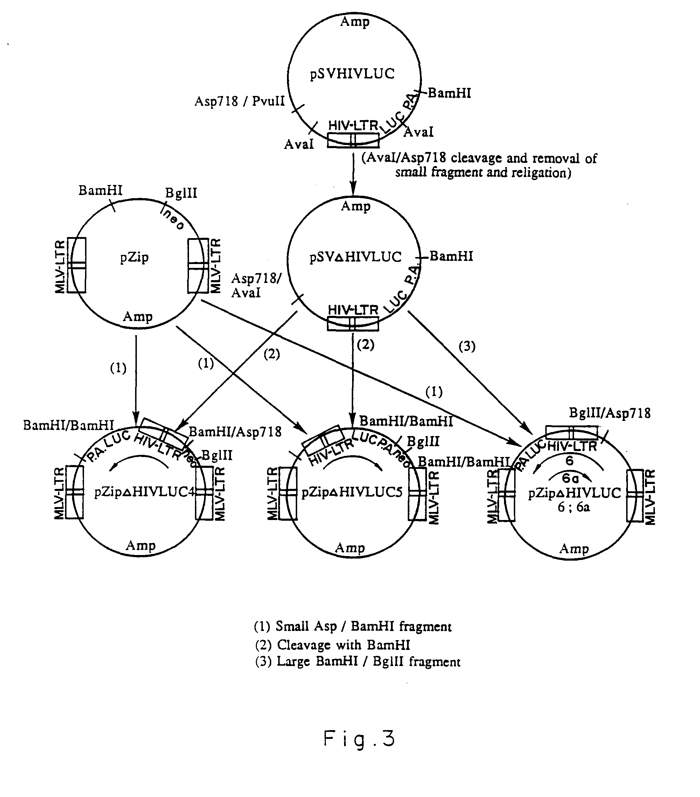

The expression of HIV is regulated by both positive and negative regulating proteins. It has been found (**12**), that the negative regulatory proteins interact at the 5' end of the LTR-DNA sequence upstream of the AvaI cleavage site. Accordingly, similar constructs to the pZipHIVLUC series but in which the sequence of the HIV-LTR upstream of the AVaI site had been deleted, were prepared. The deletion and cloning which is schematically shown in Fig. 3 was carried out as follows:

Plasmid pSVHIVLUC was cleaved with AvaI, which cleaves once at the HIV-LTR and once in the *luc* gene. Additionally, this plasmid was also cleaved with ASP718, which cleaves once in the reconstructed ASP718 site, resulting from the fusion between pVUII and ASP718 in pSVLUC12. The DNA fragment located between the ASP718 and the AvaI cleavage site was removed. The two remaining DNA fragments were ligated together, the incompatible ASP718 and AvaI cohesive ends were filled in using DNA polymerase Klenow fragment and ligated again. Following screening by restriction enzyme analysis, clones harbouring plasmids with the AVaI fragment inserted in the right orientation were selected.

The so-produced plasmid, pSVΔHIVLUC, was used in order to prepare the pZipΔHIVLUC4, 5, 6 and 6a. The strategy of the preparation of the plasmids was similar to that of the pZipHIVLUC plasmids. Briefly, the preparation was carried out as follows:

The ligation of the filled in AvaI site in pSVHIVLUC to filled in Asp718 restriction site reconstructs the Asp718 cleavage site. The plasmid was thus cleaved with Asp718 and BamHI restriction enzymes and the so-produced ASP718-BamHI DNA fragment which contains the 160 bp deleted HIV-LTR, the *luc* gene and the SV40 poly A signal and site, was isolated. This fragment was ligated into either the BamHI cleavage site of pZip in both the sense and antisense orientation relative to MLV-LTR directed transcription, thus obtaining pZipΔHIVLUC4 and pZipΔHIVLUC5, respectively, or ligated between the BamHI and the BglII cleavage sites in the antisense or sense orientation relative to MLV transcription thus obtaining plasmids pZipΔHIVLUC6 and pZipΔHIVLUC6a, respectively.

As known (**13**) the PolyA signal of the cloned firefly luciferase is inefficient. By isolating the HIV-LTR/*luc*/DNA fragment from pSVLUC12, the PolyA signal and the PolyA site were added at the 3' end of the *luc* gene and since these 3' processing signals are highly efficient it facilitates efficient generation of mRNA.

In the pZipHIVLUC1, 1, 3 and 3a and pZipΔHIVLUC4, 5, 6 and 6a, the HIV-LTR promoter occlusion is ensured by the 5'-MLV-LTR and thus the background expression of luciferase is much lower than in the plasmid pALUCAT. However, out of these 8 plasmids, those in which the HIV-LTR/*luc*/Poly A was inserted in the antisense orientation, namely plasmids pZipHIVLUC1 and 3, and pZipΔHIVLUC4 and 6, are advantageous over the others. In the latter plasmids, the MLV directed transcription which may bring about production of MLV genomic RNA is truncated by processing at the HIV-LTR processing signal.

Plasmids prepared in accordance with the present invention or used in the preparation of these plasmids are listed in the following Table 1:

## Table 1

### LIST OF PLASMIDS

| Plasmid | Source | Relevant Properties |
|---|---|---|
| pSVLUC-G | J.Collins, Germany | SV40 promoter fused to *luc* gene |
| pRSVTAT | J.Collins, Germany | HIV *tat* gene fused to RSV-LTR |
| pACAT | BTG, A. Panet | *cat* gene fused to HIV-LTR |
| pBR322 | Bolivar, F. et al. 1977 (**9**) | $Amp^R$ gene |
| pBRLUC | This work | See text |
| pALUCCAT | This work | See text |
| pSV$_2$CAT | Gorman et al. 1982 (**10**) | |
| PSV$_2$NEO | Southern et al. 1982 (**14**) | |
| pSVHIVLUC | This work | See text |
| pSVΔHIVLUC | This work | See text |
| pZIP | Cepko, C.L. et al. 1984 (**11**) | |
| pZiPHIVLUC-1 | This work | HIV-LTRLUC insert at the BamHI site in opposite orientation to MLV transcription |
| pZiPHIVLUC-2 | This work | Same as pZiPHIVLUC-1, but the HIV-LTRLUC are inserted in the same orientation as MLV transcription |
| pZiPHIVLUC-3 | This work | HIV-LTRLUC inserted in the opposite orientation to the MLV transcription between BglII-BamHI |
| pZiPHIVLUC-3a | This work | Same as pZiPHIVLUC-3, but the HIV-LTRLUC inserted in the same orientation as MLV transcription |

## Table 1 (continued)

### LIST OF PLASMIDS

| Plasmid | Source | Relevant Properties |
|---------|--------|---------------------|
| pZiPΔHIVLUC-4 | This work | Same as pZiPHIVLUC-1, but the HIV-LTR is deleted 160 bp in the 5' end |
| pZiPΔHIVLUC-5 | This work | Same as pZiPHIVLUC-2, but the HIV-LTR is deleted 160 bp in the 5' end |
| pZiPΔHIVLUC-6 | This work | Same as pZiPHIVLUC-3, but the HIV-LTR is deleted 160 bp in the 5' end |
| pZiPΔHIVLUC-6A | This work | Same as pZiPHIVLUC-3a, but the HIV-LTR is deleted 160 bp in the 5' end |

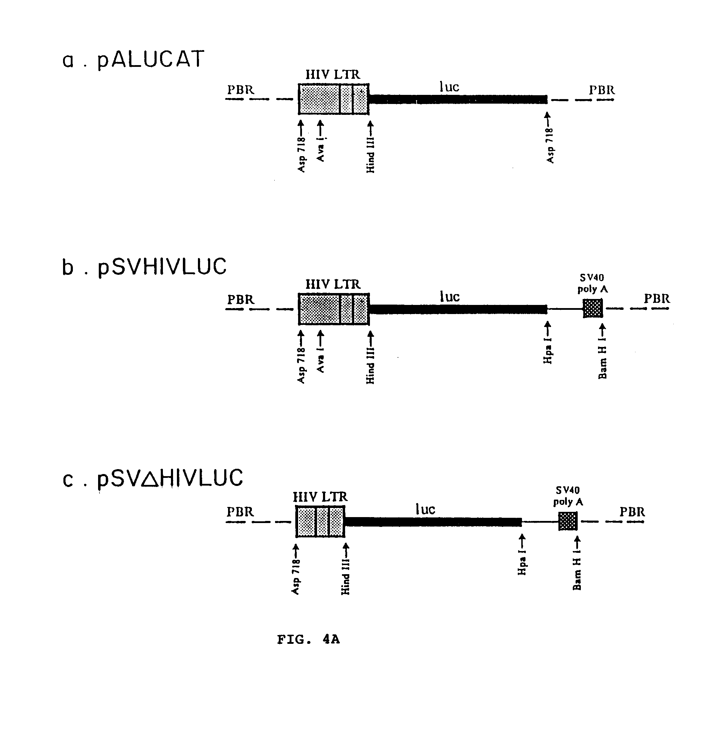

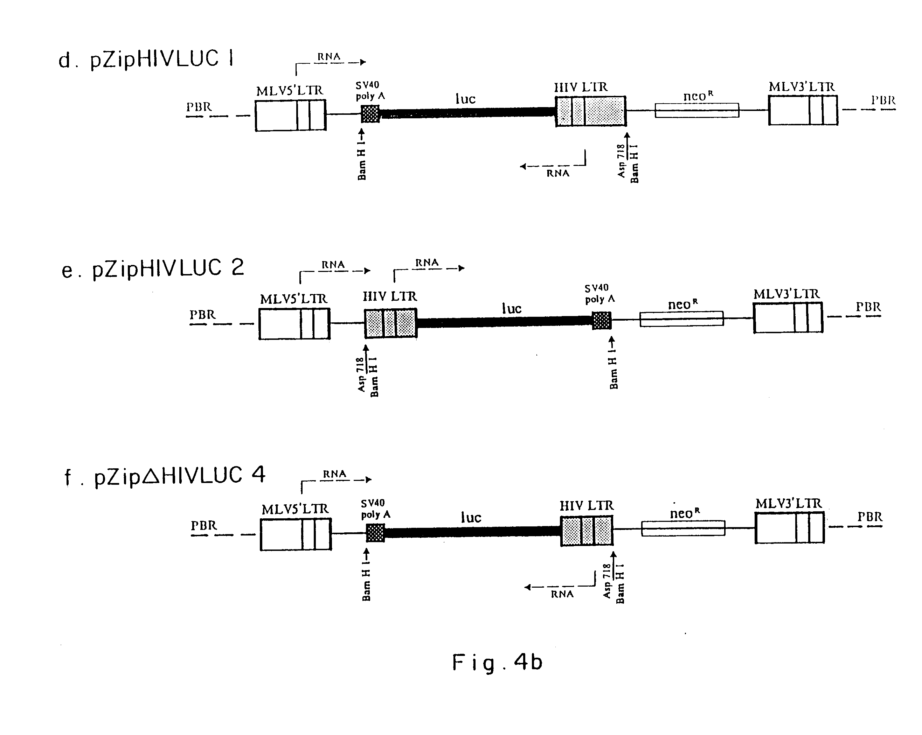

The HIV-LTR/luc region of some of the plasmids used in the following experiments is shown in Fig. 4.

**CELL LINES**

Human 293 cell line was stably transfected with pALUCAT and the gene copy number was amplified by methatroxate selection (see **15** for experimental details). These transfected cells were then infected by a CD4 carrying amphotropic retrovirus vector made in PA317 cell line following transfection with $T_4$-pMV7 (**16, 6**) and double clones were selected by neo$^R$ phenotype. The presence of the CD4 receptor was verified by anti-CD4 antibodies. The presence of the *luc* gene was verified by light measurements.

In a similar manner also 293 cells were transfected with pZipHIVLUC1-3a and pZipΔHIVLUC4-6a plasmids.

**MONITORING EXPRESSION OF LUCIFERASE BY LIGHT PRODUCTION**

In the following, some experiments are described in which the expression of luciferase in cells has been determined by monitoring light production. The procedures for monitoring light production were as follows:

Procedure A: Detection of light production in tester cell lysate

After incubation of the tester cells in accordance with the various experimental procedures, the cells were harvested, rinsed with PBS and extracts thereof were made by adding a lysis buffer (100 $\mu$l per $10^6$ cells), consisting of 1% Triton X-100, 1% BSA, 15% glycerol, 1mM DTT, 8mM MgCl$_2$, 1mM EDTA and 25mM Tris acetate pH 7.8. 10 $\mu$l of the extracts were then mixed with 250 $\mu$l of the lysis buffer, 4 $\mu$l of 40 mM ATP and then placed within a scintillation vial in a Packard liquid scintillation counter adjusted for chemiluminiscence measurements. 80 $\mu$l of 2 mM luciferin solution (Boehringer) was then added and the degree of light production in counts per minute (C.P.M.) was measured.

Procedure B: Detection of light production in whole tester cells.

Light emission from whole living tester cells was measured by a light detection camera (LDC) device. The LDC device consisted of a polaroid film cassette fitted with a sliding shutter and with a light-tight black box having a cover which allowed free flow of air but no light entry.

The tester cell cultures, in dishes, microtiter plates or chamber slides were fitted into holders which kept the bottom of the dish, plates or slides in direct contact with the polaroid film. Luciferin at a final concentration of 0.3mM was then added to the growth medium and the LDC was placed in a CO$_2$ incubator

11

and the shutter was opened for desired intervals.

**EXPERIMENTAL RESULTS**

**Example 1:** Production of light by transiently transfected cells

1 x $10^6$ cells of various sources were transfected by incubating the cells with 10 $\mu$g of a plasmid in the presence of calcium phosphate (**10**). The cells were then incubated for 24 hours, after which the cells were harvested, extracts thereof prepared and the production of light monitored in accordance with Procedure A. The basal light activity in the transfected cells is shown in the following Table 2:

<div align="center">

**Table 2**

</div>

| Plasmid | Transfected cells | C.P.M. |
|---------|-------------------|--------|
| pSVLUC-G | COS 7 | $1.4 \times 10^5$ |
| pSVLUC-G | RAT 1 | $1.2 \times 10^3$ |
| pSVLUC-G | 293 | $1 \times 10^6$ |

While the results in the above Table are not results obtained in experiments carried out in accordance with the present invention, they demonstrate that the 293 expresses luciferase considerably better than the two other cells as evidenced by the much higher light count. For this reason, the 293 cells were chosen for further experiments conducted in accordance with the invention.

**Example 2:** Enhancement of light generation by transactivation with *TAT* following cotransfection of pALUCAT and pRSVTAT

10 $\mu$g of pALUCAT and pRSVTAT were transiently transfected into 293 cells. Extracts were made 24 hours following transfection and light was measured in accordance with the above procedure A.

In an additional series of experiments, 293 cells were transfected with pALUCAT and pSV2NEO in a ratio of 1:8, respectively. The cells were exposed after transfection to G418 for a period of 2 weeks. G418 resistant colonies were suspended and the pool of resistant cells was used for transient transfection with pRSVTAT. Similarly as above, extracts were made 24 hours following transfection and light was measured in accordance with Procedure A.

The results of the two series of experiments are shown in the following Table 3:

<div align="center">

**Table 3**

</div>

| pALUCAT | pRSVTAT | C.P.M. |
|---------|---------|--------|
| Transient | – | $7 \times 10^4$ |
| Transient | + | $5 \times 10^6$ |
| Stable | – | $2 \times 10^3$ |
| Stable | + | $7 \times 10^3$ |

These results show that in the presence of the TAT, which comes from expression of the tat gene present in pRSVTAT, the production of light is enhanced.

**Example 3:** Transactivation of pALUCAT HIV-LTR by cell extracts made of 293 cells transiently transfected with pRSVTAT

1 x $10^6$ 293 cells were transfected with 5-10 $\mu$g of pALUCAT. Another group of 293 cells was transfected with pRSVTAT. 24 hours following transfection, the pRSVTAT transfected cells were lysed and cell extract was added to the pALUCAT transfected cell culture for 24 hours. Light emission was measured on cell extracts by the method of Procedure A and the results obtained in two different experiments are shown in the following Table 4:

<div align="center">

**Table 4**

</div>

| pRSVTAT transfected cell extracts | Chloroquin* | C.P.M. Experiment 1 | Experiment 2 |
|---|---|---|---|
| – | – | – | $2.4 \times 10^4$ |
| – | + | $8 \times 10^4$ | $4.7 \times 10^4$ |
| + | – | $9.2 \times 10^4$ | $4.1 \times 10^4$ |
| + | + | $2.8 \times 10^5$ | $7.3 \times 10^4$ |

(*) Chloroquin was reported to enhance absorption of the TAT protein (**5**).

The results show that pALUCAT transfected cells increase their bioluminescence in the presence of TAT in the medium, which resulted in this experiment from the pRSVTAT transfected cell extracts.

**Example 4:** Transactivation of *luc* gene of pALUCAT by HIV infected cell extract

T cells isolated from 30 ml blood of a healthy human donor were infected with HIV particles. Two weeks later, the cells were lysed by freezing and thawing and then by a Downs homogenizer. By a similar procedure, extracts were also prepared from non-infected human T cells.

The so-prepared cell extracts were then added to 293 cells transiently transfected with pALUCAT. 24 hours following the addition of the extracts, the 293 cells were harvested, lysed and light emission was measured as described in Procedure A above. The results of light production are shown in the following Table 5:

<div align="center">

Table 5

| Cell extract | C.P.M. |
|---|---|
| T cells | $5 \times 10^4$ |
| HIV infected T cells | $5 \times 10^5$ |
| No extracts | $1 \times 10^5$ |

</div>

It may be seen from the above results that exposure of the transfected cells to an extract of HIV infected T cells resulted in a considerable increase of light production.

**Example 5:** Light emission from cells transfected by two different constructs

293 cells were transiently transfected with either the plasmid pSVΔHIVLUC or pZipΔHIVLUC4 (5$\mu$g

DNA/dish) or transiently cotransfected by one of these two plasmids together with pRSVTAT. Light expression was minitored either on cell extracts by the method of procedure A, or on whole cells by the method of procedure B.

The results obtained by measuring a light emission in cell extracts is shown in the following Table 6:

## Table 6

| Plasmid | Plasmid Alone | Cotransfection with pRSVTAT | Increase after cotransfection (folds) |
|---|---|---|---|
| pSVΔHIVLUC | 2-7 x $10^4$ * | 5 x $10^6$ | x 100 |
| pZipΔHIVLUC4 | 3-3.5 x $10^3$ * | 1 x $10^7$ | x 3,000 |

*Range difference in various experiments

An can be seen, the basal light production in pZipΔHIVLUC4 transfected cells was lower than that of the pSVΔHIVLUC transfected cells and, further, the increase in light production after cotransfection is much more pronounced in the former. These results thus clearly demonstrate the advantage of using a plasmid such as pZipΔHIVLUC4 in which the HIV-LTR promoter is occluded by the 5'MLV-LTR, over the use of a plasmid such as pSVΔHIVLUC in which no such occlusion occurs.

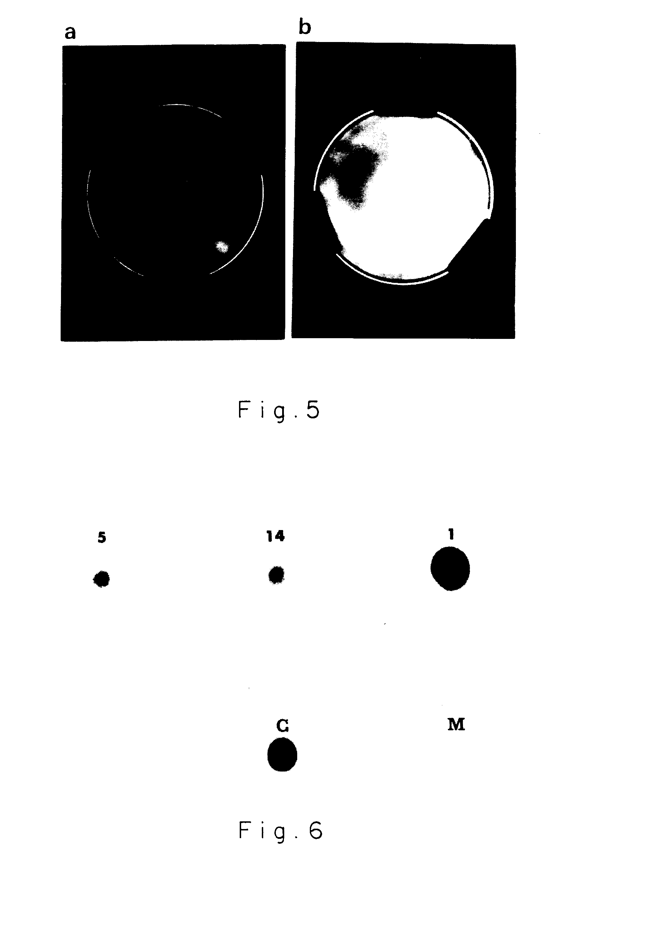

The advantage of the pZipΔHIVLUC4 plasmid is further demonstrated in Fig. 5, which shows light expression from whole cells. As can be seen in Fig. 5a, the basal light production of cells transiently transfected with pzipΔHIVLUC4 was much lower than the light production from cells transfected with pSVΔHIVLUC (Fig. 5b).

Furthermore, the results in Fig. 5 demonstrate that monitoring light production *in vivo* reliably reflects the luciferase activity as measured directly in cell extracts.

**Example 6:** Correlation between *tat* expression and light production in tester cells

293 cells were cotransfected with pRSVTAT and with pSV2NEO at a molar ratio of 10:1. The neomycin resistant clones were selected by the use of the neomycin analog, G418 (Sigma Co., U.S.A). In order to identify the *tat* expressing clones, total RNA was obtained from each of the tester's G418 resistant clones, purified by the guanidium isothiocyanite-lithium chloride method (**17**). Equal amount of RNA was then spotted on a nitrocellulose filter and hybridized to the pRSVTAT plasmid (**18**). The results from three such clones, which were arbitrarily designated as 293/*tat*1, 293/*tat*5 and 293/*tat*14, is shown in Fig. 6, wherein the upper row presents results from the dot-hybridization of clones 293/*tat*1, 293/*tat*5 and 293/*tat*14, the lower centre dot (C) represents positive control-hybridization with a spotted pRSVTAT and the lower right corner (M) contained a dot of a negative control of RNA isolated from mock transfected 293 cells.

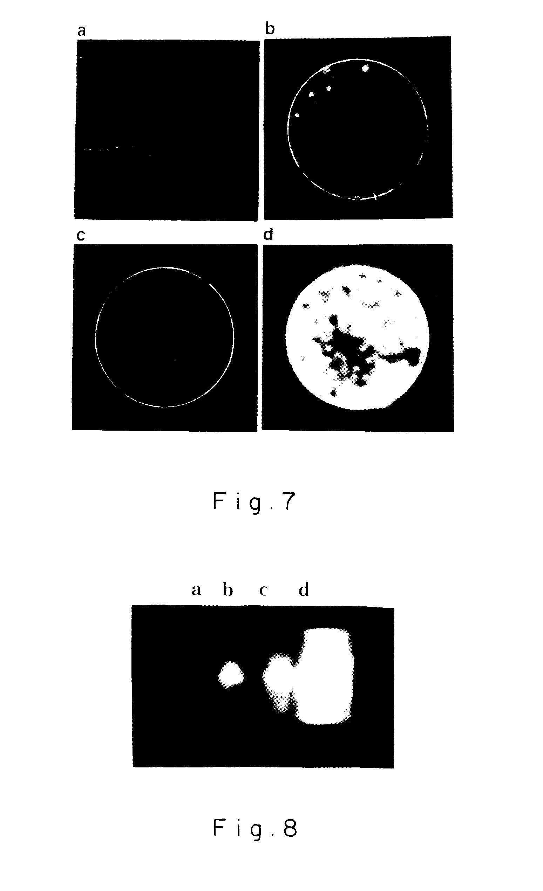

$10^5$ cells from two clones, *tat*1 and *tat*14 were then transfected with 1μg of pZipΔHIVLUC4 DNA. 24 hours following transfection, luciferin was added and light emission was monitored in the living cell in accordance with the method of Procedure B. The results are shown in Fig. 7, in which a and c and b and d are the clones 293/*tat*14 and 293/*tat*1, respectively, a and b - 5 hours of exposure, c and d - 17 hours of exposure.

It can be clearly seen that the light emission result remarkably correlates to the amount of specific *tat*-RNA detected by the dot-hybridization. No light emission was detected in 293/*tat*1 cells not transfected with pZipΔHIVLUC4 (results not shown). Microscopic observations of the culture plates indicated that the light spots corresponded to aggregates of two to ten cells. This shows that the method of light detection in accordance with Procedure B may reveal *luc* activity of only one or a few cells.

**Example 7:** Transactivation by *tat*-producing cell extracts

293 cells were transiently transfected with pZipΔHIVLUC4 and grown in a four chamber slide, 5 x $10^4$ cell/chamber. Equal aliquots of extracts of $10^5$ cells of each of the clones 293/*tat*1, 293/*tat*5 and 293/*tat*14

(see Example 6) and of a control clone, 293/pRSV, transfected with the pRSV plasmid (similar to the pRSVTAT but not containing the *tat* gene), were added each into one chamber and light emission was monitored in accordance with Procedure B. The light emission after exposure to extracts from the clone 293/PRSV, 293/*tat*5, 293/*tat*14 and 293/*tat*1 is shown in Fig. 8a, b, c and d, respectively. As can be seen when comparing the results to those of Fig. 6, the light emission after transactivation correlates remarkably with the degree of expression of the *tat* gene in the extracted cells.

**Example 8:** Transactivation of *luc* gene in cells stably transfected with pZipΔHIVLUC4

293 cells were co-transfected with pZipΔHIVLUC4 and with pSV2NEO. Independent G418 resistant clones harbouring the pZipΔHIVLUC4 plasmid were identified by light emission when grown in a multi-vale titration plate in the presence of luciferin, by the method of Procedure B. A clone designated 293/*luc*34, which exhibited low light emission in the absence of TAT, was chosen for further experimentation.

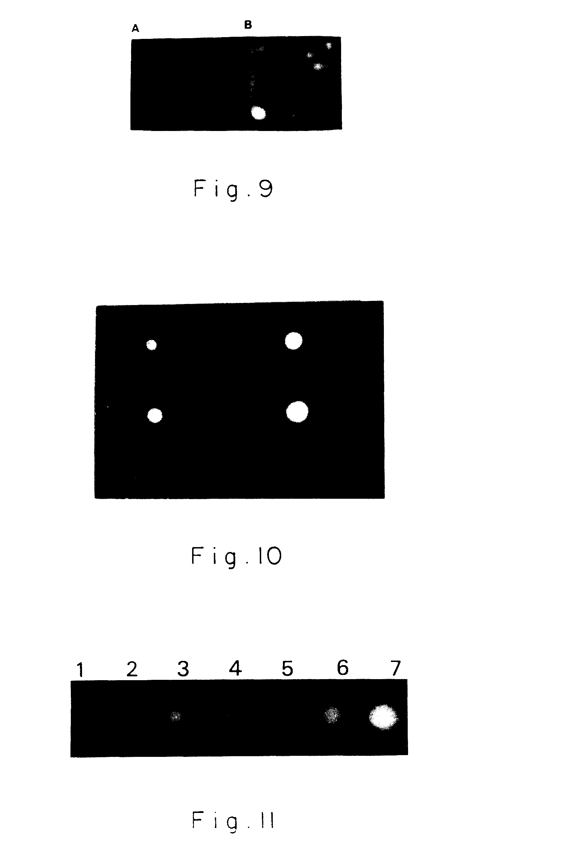

The 293/*luc*34 cells were incubated in a double chamber slide containing an equal number of cells in each chamber. Extracts from 293 cells transfected with the pRSVTAT plasmid were added to one chamber, and extracts of normal, non-transfected, 293 cells were added to the other. 2 hours later, luciferin was added and light emission was determined in accordance with the method of Procedure B. The results are presented in Fig. 9, which clearly demonstrates a considerable increase in light emission when the 293/*luc*34 tester cells were incubated in the presence of the cell extracts from pRSVTAT transfected 293 cells.

It should be noted that the time of exposure after addition of luciferin depends to a great extent on the density of the tester cells and may thus be reduced considerably by their concentration. When the cells were pelleted by centrifugation in transparent test tubes, the exposure time necessary in order to record light emission by the method of Procedure B was reduced to just a few minutes. The results of light emission from four 293/*luc*34 cell preparations concentrated in this manner, after 2 mins. of exposure, is shown in Fig. 10.

Thus, the monitoring of light production by whole, living tester cells, in accordance with the method of Procedure B, provides an easy and quick method for the identification of the presence of TAT in a medium.

**Example 9:** Transactivation of *luc* gene in various plasmids by exposure to a TAT containing medium

In a similar manner to that described above, 293 cells were transfected with various plasmids containing the *luc* gene. Light production was monitored on tester cell extracts in accordance with the method of Procedure A. Basal light production in these cells was determined and then the cells were exposed to a medium containing the TAT protein and the increase of light production following such exposure was measured. The results are shown in the following Table 7:

### Table 7

| Plasmid | Basal light production (C.P.M.) | Light production after exposure to TAT (C.P.M.) | Increase after exposure to TAT (folds) |
|---|---|---|---|
| pZipHIVLUC2 | 3,900 | 524,000 | x 134 |
| pZipHIVLUC1 | 3,500 | >1,270,000 (*) | >x 363 |
| pSVHIVLUC | 20,000 | 236,000 | x 12 |
| pSVΔHIVLUC | 1,850 | 30,000 | x 16 |

(*)  The light production exceeded the level which could be measured by the instruments.

These results clearly show that tester cells in accordance with the invention are sensitive to the presence of the TAT proteins in a tested sample and thus may be used for the diagnosis of AIDS. The results further show that tester cells containing plasmid in which the HIV promoter occlusion is ensured by the presence of a 5'-MLV-LTR show a much higher difference between the test and the background light, as compared to tester cells containing plasmids in which the promoter is not occluded (pSVHIVLUC and pSVΔHIVLUC).

**Example 10:** Cotransfection of cells with pZipHIVLUC1 and pRSVTAT

Three cultures of 293 cells were divided each into a control and a test culture. 5μg of pZipHIVLUC1 DNA was added into each control or test culture and various amounts of pRSVTAT were added into each of the test cultures. After 30-40 hrs. of incubation, the production of light was monitored on the extracts of the tester cells in accordance with the method of Procedure A as described above and the results (expressed in light units) are shown in the following Table 8:

### Table 8

| Culture | pRSVTAT | Light Units |
|---------|---------|-------------|
| 1 | 5 μg | $1 \times 10^7$ |
|   | – | 3,000 |
| 2 | 1 μg | $1 \times 10^7$ |
|   | – | 2,800 |
| 3 | 0.1 μg | $2 \times 10^6$ |
|   | – | 3,100 |

The test culture of culture 3 was infected with 50 folds less pRSVTAT than the test culture of culture 1 and thus the TAT production in this culture was considerably less. Nevertheless, the expression of luciferase, as evidenced by the light measurement, was only slightly reduced. This clearly shows that the test in accordance with the invention is effective in detecting even minute quantities of TAT.

**Example 11:** Transactivation of tester cells by extracts of T cells chronically infected with HIV

$10^4$ 293/*luc*34 cells were inoculated into each of 7 wells and then 20μl of extract, of $10^6$ HUT78 cells (**19**) was added to one of the wells, and tenfold dilutions of equal volumes were added to each of five of the other six wells (down to $10^{-5}$ dilution). In one well, a non-infected cell extract was added as a control. Light emission was detected by the method of Procedure B and the results are shown in Fig. 11.

As can be seen, light was emitted from all the test wells while no light emission was seen in the control well (the left well). These results also demonstrate the ability to detect very minute quantities of the TAT protein.

**Example 12:** Detection of HIV *tat* in blood of patients

Lymphocytes from blood samples of HIV sera positive and random blood donors were separated by standard density gradient centrifugation. About $10^4$ - $10^6$ cells were lysed by freezing and thawing and then the lysate was diluted ten-thousandfold and 20μl were added to wells containing $10^4$ 293/*luc*34 cells. Light emission was tested by the method of Procedure B.

While no light emission was detected when normal blood samples were tested, all extracts from HIV sera positive blood caused light emission from the 293/*luc* cells.

REFERENCES

1. Ratner et al., (1987). AIDS Research and Human Retroviruses, 3, 57-69.

2. Sodorski et al., (1985). Science, 229, 74-77.

3. Hauber et al., (1988). J. Virol. 62, 673-679.

4. Jakobovits et al., (1988). Mol. Cell Biol. 8, 2555-2561.

5. Frankel et al., (1988). Cell, 55, 1189-1193.

6. Feber et al., (1988). Science, 239, 184-187.

7. Garcia et al. (1987). EMBO, 6, 37651-3770.

8. Connelly et al., (1989). Cell, 57, 561-571.

9. Bolivar et al., (1977). Gene 2, 95-13.

10. Gorman et al., (1982). Mol. Cell Biol., 2, 1044-1051.

11. Cepko et al., (1984). Cell, 37, 1053-1062.

12. Garcia et al., (1987). EMBO, 6, 3761.

13. De Wet et al., (1987). Mol. Cell. Biol., 7, 725-737.

14. Southern et al., (1982). J. Mol. Appl. Genet., 1, 377-341.

15. Simonsen et al., (1983). Proc. Natl. Acad. Sci. U.S.A., 80, 2495-2499.

16. Maddon et al., (1986). Cell, 47, 333-348.

17. Chirguin et al., (1979). Biochemistry 18, 5294-5299.

18. Maniatis et al., (1982). A laboratory manual, Cold Spring Harbor Laboratory, Cold Spring Harbor, New York, U.S.A.

19. Gasdar et al., (1980). Blood 58, 409-417.

## Claims

1. A method for the diagnosis of HIV infection in a suspected individual, comprising:

   a) providing tester cells transfected by a recombinant DNA molecule having

   i) an HIV TAT-inducible promoter selected from the group consisting of an HIV-LTR or part thereof including the *tar* site or an analogous sequence capable of inducing the expression of a downstream DNA sequence in the presence of HIV TAT,

   ii) a heterologous DNA sequence encoding an enzyme capable of catalysing a reaction by which light is emitted, said sequence being under the expression control of said promoter, said tester cells capable of absorbing a TAT protein from a surrounding medium and/or susceptible to HIV infection;

   b) contacting said tester cells with a blood sample from the suspected individual or with an appropriate fraction thereof and subjecting the tester cells or extracts thereof to conditions in which expression of said heterologous DNA sequence will result in light emission; and

   c) monitoring light emission by said cells or extracts thereof, the detection of light emission above background levels indicating an HIV infection.

2. The method according to Claim 1, wherein said tester cells are susceptible to HIV infection and are contacted with a blood sample or a fraction thereof containing either free HIV particles if present in the blood, $T_4$ helper cells, or both.

3. The method according to Claim 2, comprising contacting said cells with the blood sample or said fraction thereof in the presence of a fusing or a transport enhancing agent.

4. The method according to Claim 1, wherein said tester cells are capable of absorbing the TAT protein from a surrounding medium, and are contacted with a blood sample or a fraction thereof containing the TAT protein if present in the blood.

5. The method according to Claim 4, wherein said fraction of the blood comprises a lysate of $T_4$ helper cells.

6. The method according to any one of the preceding claims wherein said tester cells are susceptible to HIV infection and absorption of the TAT protein from the surrounding medium.

7. The method according to any one of the preceding claims, wherein the said tester cells are derived from the 293 cell line.

**8.** The method according to any one of the preceding claims, wherein monitoring light emission is performed on whole tester cells preparations.

**9.** The method according to any one of Claims 1 to 7, wherein monitoring light emission is performed on extracts of tester cells.

**10.** A recombinant DNA molecule comprising an HIV promoter, said promoter being an HIV-LTR or part thereof including the tar site or an analogous sequence capable of inducing the expression of a downstream DNA sequence in the presence of HIV TAT, and a heterologous DNA sequence encoding an enzyme catalysing a light-emitting reaction, said DNA sequence being under the expression control of said promoter.

**11.** The recombinant DNA molecule according to Claim 9, wherein said heterologous DNA sequence encodes the enzyme luciferase.

**12.** The recombinant DNA molecule according to Claim 11, comprising at its 3' end also a different poly A site and sequence than that of said heterologous DNA sequence.

**13.** The recombinant DNA molecule according to Claim 12, which is the plasmid pALUCAT, having the restriction map given in Fig. 1, or pSHIVLUC, having the restriction map given in Figure 2.

**14.** The recombinant DNA molecule according to any one of Claims 10 to 12, wherein said HIV promoter is downstream to another promoter, which is more active than the HIV promoter in the absence of TAT.

**15.** The recombinant DNA molecule according to Claim 14, wherein said promoter is in the antisense orientation relative to the direction of transcription induced by said other promoter.

**16.** The recombinant DNA molecule according to claim 15, which is the plasmid pZipHIVLUC1, having the restriction map given in Figure 2, or pZipΔHIVLUC4, having the restriction map given in Figure 3.

**17.** The recombinant DNA molecule according to Claim 14, wherein said promoter is in the sense orientation relative to the direction of transcription induced by said other promoter.

**18.** The recombinant DNA molecule according to claim 17, which is the plasmid pZipHIVLUC2, having the restriction map given in Figure 2, or pZipΔHIVLUC5, having the restriction map given in Figure 3.

**19.** A method according to any one of Claims 1 to 9, wherein said recombinant DNA molecule is a molecule according to any one of Claims 10 to 18.

**20.** A cell transfected by a DNA molecule according to any one of Claims 10 to 18.

**21.** A process for preparing cells useful in the method according to Claim 1, comprising transfecting cells with a DNA molecule according to any one of Claims 10 to 18, the cells to be transfected or selected from the group consisting of;
i) cells which are *a priori* capable of absorbing a TAT protein from this surrounding medium;
ii) cells which are *a priori* susceptible to HIV infection; and
iii) cells which are cotransfected with a gene encoding the CD4 receptor.

**22.** Tester cells prepared by the process of Claim 21.

**23.** A kit for carrying out the method according to any one of Claims 1 to 9, comprising transfected cells according to Claims 20 or 22.

**24.** A system for carrying out the method according to any one of Claims 1 to 9, comprising transfected cells according to Claims 20 or 22 and a light monitoring apparatus.

Fig. I

(1) Small Asp718 / BamHI fragment
(2) Cleavage with BamHI
(3) Large BamHI / BglII fragment

Fig.2

Fig.3

FIG. 4A

a . pALUCAT

b . pSVHIVLUC

c . pSVΔHIVLUC

22

d. pZipHIVLUC I

e. pZipHIVLUC 2

f. pZip△HIVLUC 4

Fig. 4b

a                   b

Fig.5

5              14              1

C                   M

Fig.6

a          b

c          d

Fig.7

a   b   c   d

Fig.8

Fig.9

Fig.10

Fig.11

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | GENE, vol. 88, 16th April 1990, pages 197-205, Elsevier Science Publishers B.V. (Biomedical Division); O. SCHWARTZ et al.: "A microtransfection method using the luciferase-encoding reporter gene for the assay of human immuno-deficiency virus LTR promoter activity" * Whole document * | 1-24 | C 12 Q 1/66 C 12 N 15/85 C 12 Q 1/70 G 01 N 33/569 |
| | — — — | | |
| D,A | WO-A-8 903 878 (INSTITUT PASTEUR) | | |
| | — — — | | |
| D,A | SCIENCE, vol. 239, 1988, pages 184-187; B.K. FELBER et al.: "A quantitative bioassay for HIV-1 based on trans-activation" | | |
| | — — — — — | | |

TECHNICAL FIELDS SEARCHED (Int. Cl.5)

C 12 Q

The present search report has been drawn up for all claims

| Place of search | Date of completion of search | Examiner |
|---|---|---|
| The Hague | 30 September 91 | MOLINA GALAN E. |

CATEGORY OF CITED DOCUMENTS

X : particularly relevant if taken alone
Y : particularly relevant if combined with another
document of the same catagory
A : technological background
O : non-written disclosure
P : intermediate document
T : theory or principle underlying the invention

E : earlier patent document, but published on, or after
the filing date
D : document cited in the application
L : document cited for other reasons

&: member of the same patent family, corresponding
document